# FASCICULE DE BREVET EUROPEEN

(11) **EP 1 789 568 B1**
(45) Date de publication et mention de la délivrance du brevet: **26.04.2017**
(21) Numéro de dépôt: 05798144.1
(22) Date de dépôt: 02.09.2005
(51) Int. Cl.: C12P 1/00, C12P 19/04

(54) **PROCEDE D'OBTENTION DE FRACTIONS ISSUES DU SON DE BLE AINSI QUE LES FRACTIONS AINSI OBTENUES**
VERFAHREN ZUM ERHALT VON FRAKTIONEN VON WEIZENKLEIE UND DAMIT ERHALTENE FRAKTIONEN
METHOD FOR OBTAINING FRACTIONS FROM WHEAT AND FRACTIONS THUS OBTAINED

(30) Priorité: 03.09.2004 FR 0409336
(43) Date de publication de la demande: 30.05.2007
(73) Titulaire: Bioactor B.V., 6229 EV Maastricht (NL)
(72) Inventeur: DUGENET, Yann, F-22000 Saint-Brieuc (FR)
(74) Mandataire: LC Patents
(86) Numéro de dépôt international: PCT/FR2005/050702
(87) Numéro de publication internationale: WO 2006/027529

(56) Documents cités:
- WO-A-02/02644
- WO-A-98/22613
- WO-A1-01/67891
- DONG-HO WOO: "PREPARATION OF SOLUBLE DIETARY FIBRE BY USING CORN HULL" FOOD SCIENCE AND BIOTECHNOLOGY, KOREAN SOCIETY OF FOOD SCIENCE AND TECHNOLOGY, SEOUL, KR, vol. 10, no. 2, 2001, pages 144-148, XP009048545 ISSN: 1226-7708
- YAMADA H. ET AL.: "Structure and properties of oligosaccharides from wheat bran", CEREAL FOODS WORLD, vol. 38, no. 7, July 1993 (1993-07), pages 490-492, XP001018255, ISSN: 0146-6283
- SHIKATA S. ET AL.: "Purification and Properties of an exo-Cellulase Component of Novel Type from Trichoderma virile", J. BIOCHEM., vol. 78, no. 3, 1975, pages 499-512, XP055192901,
- MAES C. ET AL.: "Relative activity of two endoxylanases towards water-unextractable arabinoxylans in wheat bran", J. CEREAL SCI., vol. 39, no. 2, February 2004 (2004-02), pages 181-186, XP027111966,

## Description

L'invention concerne un procédé d'obtention de fractions issues du son de céréales ainsi que les fractions ainsi obtenues.

Yamada H. et al., Cereal Foods World, 1993, 38(7) : 490-492 concerne l'obtention d'oligosaccharides à partir de son de blé selon le procédé suivant. Le son de blé est lavé à l'eau chaude pour enlever l'amidon et les protéines hydrosolubles, puis l'hemicellulose est extraite en milieu alkalin. Après dessalage et concentration par ultrafiltration, l'hemicellulose est hydrolysée avec la cellulase Onozuka RS. Les hydrolysats sont ensuite purifiés par passage dans une colonne de carbone et élution à l'éthanol 10-70%, concentrés, puis lyophilisés pour générer les oligosaccharides de son de blé.

Maes C. et al., J. Cereal Sci., 2004, 39(2) : 181-186 concerne le traitement de son de blé par différentes enzymes notamment des endoxylanases pour l'obtention de fractions solubles et insolubles de fibres alimentaires. Les enzymes utilisées sont entre autres la Termamyl 120L de Novozymes (α-amylase), la Neutrase 0.8L de Novozymes (protéase) et la Shearzyme 500L de Novozymes (xylanase, hemicellulase et cellulase). Le procédé d'obtention des fractions comprend une étape d'élimination de l'amidon du son de blé par action de la Termamyl 120L, suivie par une étape d'hydrolyse enzymatique avec Shearzyme 500L, puis une séparation par filtration et congélation des fractions obtenues.

WO 01/67891 concerne un procédé de fabrication de matières physiologiquement actives séparées des céréales telles que le blé, notamment l'acide férulique et l'arabinoxylane présents dans le son, qui comprend une séparation par extrusion suivi d'un traitement par des enzymes hydrolysantes de la paroi cellulaire des végétaux.L'extrudat est dispersé dans de l'eau et la paroi cellulaire est décomposée par traitement avec des hydrolases telles que cellulase ou hemicellulase, arabinase ou xylanase pour séparer l'acide férulique et l'arabinoxylane.

Il existe actuellement un grand intérêt pour des produits qui ont de fortes capacités de rétention d'eau et/ou d'huile et/ou des propriétés de structuration de la matrice dans laquelle ils sont inclus.

En particulier, dans l'industrie de la boulangerie, des produits rétenteurs d'eau sont introduits dans la pâte à cuire pour procurer, du fait de l'évaporation de l'eau qu'ils retiennent, lors de la cuisson de la pâte boulangère, le gonflant et le moelleux voulu.

Les produits rétenteurs d'eau sont actuellement utilisés dans de nombreux domaines autres que l'alimentation. Par exemple, ils sont utilisés en jardinerie, où les produits sont souvent des produits minéraux, dans le domaine de l'hygiène où on utilise principalement de la cellulose.

D'autres applications des produits rétenteurs d'eau et/ou d'huile sont dans le domaine de la cosmétique, de la parfumerie, de la pharmacie ou encore dans le domaine des produits satiétogènes.

Dans tous ces domaines, une innocuité pour la santé humaine des produits utilisés est importante.

De nombreux produits de synthèse rétenteurs d'eau et/ou d'huile existent mais ils sont souvent très coûteux en raison de leurs procédés d'obtention.

Ces produits rétenteurs d'eau sont, le plus souvent, insolubles dans l'eau.

D'autre part, l'intérêt des fibres dans l'alimentation est maintenant reconnu. Il est largement recommandé de consommer des produits alimentaires contenant des fibres, éventuellement ajoutées, qui améliorent le transit intestinal, entre autres.

Ainsi, de nombreux produits alimentaires sur le marché actuel contiennent de telles fibres ajoutées.

Pour faciliter leur consommation, ces fibres sont ajoutées dans les produits alimentaires tels que les produits laitiers, etc., sous forme de fibres solubles dans l'eau. Ici aussi, il est important que les fibres ajoutées soient sans danger pour la santé humaine et des fibres d'origine végétale sont tout à fait recommandées.

Mais, les fibres utilisées actuellement sont souvent d'origine purement synthétique et/ou très coûteuses en raison de leur procédé de fabrication.

L'invention vise à pallier les inconvénients des produits rétenteurs d'eau et d'huile et des fibres solubles dans l'eau ainsi que de leurs procédés d'obtention, de l'art antérieur, en proposant un procédé simplifié et économique d'obtention, d'une part, d'une fraction insoluble ayant des propriétés améliorées de rétention d'eau et d'huile, et d'autre part, d'une fraction soluble riche en arabinoxylanes, à partir de sons de céréales issus des procédés de meunerie et d'amidonnerie.

A cet effet, l'invention propose un procédé d'obtention de fractions issues du son de blé, pour obtenir une fraction soluble dans l'eau et une fraction insoluble dans l'eau comprenant les étapes suivantes :
a) broyage du son à une granulométrie inférieure ou égale à 250 µm ; ensuite
b) réduction du taux d'amidon par action d'une enzyme α-amylase avec un rapport en poids enzyme/matière sèche de départ variant entre 0,1% et 1,0% ;
c) hydrolyse,
d) séparation des fractions voulues, et
e) séchage des fractions séparées et obtenues caractérisé en ce que le son de blé utilisé est choisi dans le groupe constitué par les sons issus du procédé de meunerie et les sons issus du procédé d'amidonnerie, et en ce que l'étape c) d'hydrolyse est une étape d'hydrolyse enzymatique dans laquelle les enzymes utilisées ont au moins deux activités enzymatiques choisies parmi une activité xylanase et une activité cellulase, hemicellulase ou arabanase, et où l'hydrolyse est réalisée à 60°C plus ou moins 10°C et à un pH de 5 plus ou moins 1,0 ; et où le rapport en poids enzyme/matière sèche de départ est compris entre 0,1 et 0,3%.

Selon un premier mode de mise en oeuvre du procédé de l'invention, les enzymes utilisés à l'étape b) ont au moins les deux activités xylanase et cellulase.

Selon un second mode de mise en oeuvre du procédé de l'invention, les enzymes utilisés à l'étape b) ont au moins les deux activités xylanase et hémicellulase.

Selon un troisième mode de mise en oeuvre du procédé de l'invention, les enzymes utilisés à l'étape b) ont au moins les deux activités xylanase et arabanase.

Dans une première variante du procédé de l'invention, les sons sont les sons issus du procédé de meunerie.

Dans une seconde variante du procédé de l'invention, les sons sont les sons issus du procédé d'amidonnerie.

L'invention propose également une fraction extraite du son de céréales, caractérisée en ce qu'elle est insoluble dans l'eau, en ce qu'elle a une teneur en amidon inférieure à 6 % en poids par rapport à la masse sèche de la fraction totale sèche, et en ce qu'elle a une capacité de rétention d'eau supérieure ou égale à 5 g d'eau par g de fraction sèche et une capacité de rétention d'huile supérieure ou égale à 2,0 g d'huile par g de fraction sèche.

L'invention propose encore une fraction extraite du son de céréales, caractérisée en ce qu'elle est soluble dans l'eau, et elle a une teneur en arabinoxylanes supérieure ou égale à 30 % en poids par rapport à la masse sèche de la fraction totale, et en ce que les arabinoxylanes ont un rapport molaire arabinose/xylose, noté A/X compris entre 0,33 et 0,46 et en ce que les arabinoxylanes ont un degré de polymérisation, noté DP, supérieur ou égal à 3.

Les fractions selon l'invention peuvent être obtenues par le procédé de l'invention à partir du son de blé.

L'invention sera mieux comprise et d'autres avantages et caractéristiques de celle-ci apparaîtront plus clairement à la lecture de la description explicative qui suit.

Le procédé dit procédé de meunerie est un procédé de fabrication de farine à partir de céréales qui comprend en tant que première étape, un nettoyage des grains de céréales par des techniques de séparation gravitaire et de brossage, avec bien sûr une élimination des éléments métalliques ferreux. Après ce nettoyage, on obtient les grains de céréales propres débarrassés de ces déchets.

Ensuite, le grain de céréale subit une alternance de phases de broyage et ou de tamisage au cours desquelles on sépare tout d'abord les gros sons puis les sons fins. La farine obtenue à l'issue du procédé de meunerie est une farine alimentaire de différents types selon le taux d'enveloppe du grain ou son qu'elle contient encore. Actuellement, les sons issus de ce procédé, dits "sons issus du procédé de meunerie", sont soit rejetés soit faiblement valorisés en alimentation animale.

Cette étape de meunerie est la première étape du procédé dit procédé d'amidonnerie au cours duquel on procède à une séparation du gluten de la farine pour obtenir, d'une part, du gluten vital et, d'autre part, du lait d'amidon. Le lait d'amidon contient encore de petites quantités de sons de l'enveloppe de la céréale, c'est-à-dire du son mais sous forme de fibres. Le lait d'amidon est filtré pour obtenir, d'une part, le lait d'amidon que l'on récupère et, d'autre part, le son qui est sous forme de fibres et que l'on appellera ici "son issu du procédé d'amidonnerie". Ce son est actuellement lui aussi soit rejeté soit faiblement valorisé en alimentation animale.

Autrement dit, au cours du procédé d'amidonnerie qui comprend, en tant que premières étapes, les étapes du procédé de meunerie, on sépare l'enveloppe du grain de céréale, ou son, du grain lui-même. Cette enveloppe est constituée, d'une part, de l'ensemble des couches du péricarpe qui sont les couches externes de l'enveloppe et, d'autre part, de la couche aleurone dite couche AL qui est une des couches internes de l'enveloppe.

Ainsi, lors du procédé de meunerie, on sépare la plus grande partie de l'enveloppe du grain, que l'on appellera ici "son issu du procédé de meunerie" qui est essentiellement constitué de l'ensemble des couches du péricarpe et partiellement de la couche aleurone et, le son qui est sous forme de fibres, que l'on appellera ici "son issu du procédé d'amidonnerie", qui est lui issu exclusivement des couches internes de l'enveloppe à savoir principalement de la couche aleurone AL.

On a maintenant découvert qu'en traitant ces deux types de sons selon le procédé de l'invention, on obtient en sortie du procédé deux fractions issues du son de céréales qui présentent, pour l'une un intérêt en tant que produit rétenteur d'eau et d'huile, et pour l'autre, une solubilité et une coloration blanche intéressantes pour une utilisation en particulier dans l'industrie agroalimentaire.

Ces sons issus du procédé de meunerie ou du procédé d'amidonnerie contiennent environ 50 % en poids d'amidon par rapport à la masse sèche totale des sons.

Les sons issus du procédé de meunerie ont une granulométrie grossière : 80 % des particules ont un diamètre moyen supérieur à 350 µm alors que les sons ou fibres issus du procédé d'amidonnerie ont une granulométrie plus faible : 50 % des particules ont un diamètre moyen inférieur à 250 µm.

La première étape du procédé de l'invention consiste donc à broyer les sons de départ à une granulométrie inférieure ou égale à 250 µm.

La granulométrie est mesurée par passage des sons au travers de tamis vibrants ayant différentes ouvertures de mailles et pesée des fractions passantes et refusées, selon la norme 965-22 (méthode de type I avec spécification des tamis selon la norme ISO 3310/1, 1982).

Ensuite, les sons issus du procédé de meunerie ou du procédé d'amidonnerie sont traités pour réduire le taux d'amidon qu'ils contiennent et ainsi enrichir en fibres les produits finis. Cette étape dite de traitement de l'amidon est effectuée par action d'un enzyme α-amylase à activité majoritairement α-amylasique avec un rapport en poids enzyme / matière sèche de départ variant de préférence entre 0,1 % et 1,0 %. L'amidon est ensuite séparé du son mécaniquement par pressage, ou tamisage ou filtration frontale ou tangentielle, comme cela apparaîtra le plus avantageux à l'homme de l'art.

La teneur en amidon est mesurée par la méthode EWERS, 3^{ème} Directive CEE 72/199 rectifiée par JOCE du 27 novembre 1980.

Puis les sons de céréales obtenus sont hydrolysés pour séparer la fraction soluble de la fraction insoluble des sons de céréales. L'hydrolyse est réalisée à 60 °C plus au moins 10 °C et à un pH de 5 plus ou moins 1,0. Elle est réalisée avec un enzyme ayant au moins deux des activités suivantes : xylanase, arabanase, β-glucanase, cellulase, hémicellulase et pectinase. De préférence, l'enzyme aura au moins deux des activités xylanase et cellulase ou encore xylanase et arabanase ou encore xylanase et hémicellulase. Le rapport en poids enzyme / matière sèche de départ dans cette étape d'hydrolyse est de préférence compris entre 0,1 % et 0,3 %.

Ces enzymes sont couramment utilisés dans l'industrie alimentaire en amidonnerie et en glucoserie et sont acceptables pour l'alimentation humaine et animale.

A l'issue de cette étape d'hydrolyse, on obtient d'une part une fraction insoluble et d'autre part une fraction soluble que l'on sépare par décantation et clarification suivies éventuellement d'une filtration frontale ou tangentielle.

Chaque fraction séparée est ensuite séchée pour obtenir des fractions solides.

Les fractions obtenues sont une fraction insoluble et une fraction soluble. Ces deux fractions sont enrichies en fibres. Cependant, les fibres solubles sont enrichies en arabinoxylanes avec des caractéristiques de rapport arabinose/xylose, noté A/X, faible.

Ceci est particulièrement avantageux car les arabinoxylanes, dont la formule est (C₅H₈O₄)ₙ, sont constituées d'enchaînement de β 1,4 xylose substitués sur les carbones 2 et/ou 3 par des arabinoses. L'acide férulique C₆H₃(OH)(OCH₃)(C₂H₂COOH), composé phénolé du son peut être présent sur le carbone 5 de l'arabinose par une liaison ester. Le degré de substitution de l'enchaînement xylose, par des arabinoses est dénoté par le rapport entre le nombre de mole d'arabinose comparé au nombre de mole de xylose, et est noté A/X. Le ratio A/X habituellement connu est de 0,5 à 0,6, avec des valeurs extrêmes comprises entre 0,3, pour du son issu de la couche aleurone AL et de 1, pour des sons appauvris en couche AL. Le degré de substitution est croissant de la couche AL vers le péricarpe.

La teneur moyenne en arabinoxylanes, notés AX, dans les sons de blé est d'environ 25 % en poids par rapport à la matière sèche.

La teneur en arabinoxylanes est déterminée par la méthode ENGLYST, 1988, par chromatographie en phase gazeuse, CG. Dans cette méthode des étalons et des solutions témoins sont utilisés pour mesurer le facteur de réponse (FRP). Les échantillons et les témoins sont préparés selon les mêmes méthodes : extraction au solvant, acétylation, réduction, hydrolyse.

Les AX de la couche AL sont moins facilement dégradés dans le système digestif.

L'effet des AX sur la santé est lié selon certains auteurs au rapport A/X. En effet, les arabinoses sont plus facilement fermentescibles, et ce d'autant plus qu'ils sont accessibles en bout de chaîne. Au fur et à mesure de l'hydrolyse des arabinoses, le squelette xylose devient plus hydrophobe.

Un rapport A/X faible dénote une faible teneur en arabinose ce qui est avantageux car la chaîne xylose est plus hydrophobe et moins fermentescible, ce qui signifie que la fermentation se fera alors préférentiellement dans le colon.

L'effet prébiotique est lié au fait que les molécules de xylose dans leur dégradation par la flore intestinale, amènent une modification de cette flore et le développement spécifique de certains microorganismes.

L'effet prébiotique est donc d'autant plus marqué que le rapport A/X est faible.

Le rapport A/X est déterminé par mesure des concentrations en arabinoses et xyloses par la méthode ENGLYST (Englyst H.N. & Cummings J.H., J. Assoc. Anal. Chem (1998), 71, 808-814 et en faisant le rapport de ces concentrations.

De plus, cette fraction soluble est bien constituée de fibres car ses constituants ont un degré de polymérisation, noté DP, supérieur a 3.

Le degré de polymérisation DP est mesuré par chromatographie liquide haute performance ionique, HPLIC, et par chromatographie sur couche mince.

Les mesures par HPLIC sont effectuées selon le protocole suivant:

| | | |
|---|---|---|
| Colonne : | Carbopac PA 1 | |
| Détecteur : | Ampérométrie pulsée | |
| Débit : | 1 ml/min | |
| Eluant : | 1) NaOH | 150 mM |
| | 2) CH₃COONa | 800 mM |

**Gradient :**

| Temps (min) | 1) NaOH 150 mM | 2) CH₃COONa 800 mM |
|---|---|---|
| 0 | 100 % | 0 % |
| 5 | 100 % | 0 % |
| 60 | 50 % | 50 % |
| 65 | 50 % | 50 % |
| 66 | 100 % | 0 % |

| | |
|---|---|
| Témoins : | DP1 : Glucose, Xylose, |
| | DP2 : Maltose, |
| | DP3 (facultatif) : Maltotriose |

Les mesures par CCM sont effectuées selon le protocole suivant :
Les plaques sont recouvertes d'une couche de 0,2 mm de gel de silice (source : Merck Nogent/Marne).
L'échantillon à analyser et les témoins sont déposés sur la plaque.
L'éluant est un mélange de n-butanol, acide acétique et eau en rapport en volume de 2:1:1.
Durée d'élution : 4 h.
Le réactif de révélation est l'acide sulfurique concentré.

Le rapport A/X de la fraction soluble obtenue est compris entre 0,3 et 0,6, de préférence entre 0,33 et 0,46. Le rapport A/X est mesuré.

Le rendement des AX dans la fraction soluble en fin de procédé est de 10 à 15 % en poids par rapport au poids de la matière sèche de départ.

La fraction insoluble présente des capacités améliorées de rétention d'eau comprise entre 5,5 g et 6,0 g d'eau par g de fraction insoluble sèche et de rétention d'huile comprise entre 2,5 g et 2,8 g d'huile par g de fraction insoluble sèche.

De préférence, les sons issus du procédé de meunerie sont traités séparément des sons issus du procédé d'amidonnerie en raison de leurs granulométries différentes.

Cependant, dans les deux cas, l'étape essentielle du procédé est l'étape d'hydrolyse avec des enzymes ayant au moins deux des activités précédemment citées.

Afin de mieux faire comprendre l'invention, on va maintenant en décrire, à titre d'exemples purement illustratifs et non limitatifs, plusieurs modes de mise en oeuvre.

### EXEMPLE 1 : Traitement du son de blé issu du procédé de meunerie

Le produit de départ était du son de blé dont 80 % des particules avaient un diamètre équivalent moyen supérieur à 350 µm.

Les sons de blé subissent d'abord un traitement de micronisation pour obtenir un son de blé dont 50 % des particules avaient un diamètre moyen inférieur à 125 µm.

La granulométrie a été mesurée par pesée après tamisage sur tamis vibrants.

La teneur en amidon de ces sons de blé varie de 25 % à 50 % en poids par rapport au poids du son de départ sec.

On a ensuite procédé à un traitement pour séparer l'amidon du son de blé. Cela a été effectué par action d'un enzyme α-amylase TERMAMYL 120 L^{®} de NOVO qui a une activité enzymatique majoritairement α-amylasique, en réacteur agité maintenu à une température de 70 °C +/-10 °C et à un pH de 5 ± 0,5. Le rapport en poids enzyme / matière sèche de départ était de 0,1 %.

La durée du traitement était de 1 heure.

L'amidon a été rejeté et le produit restant a subi l'étape d'hydrolyse caractéristique du procédé de l'invention.

L'enzyme utilisé était le NOVOZYME 28012^{®} de NOVO. L'enzyme NOVOZYME 28012^{®} a les activités enzymatiques xylanase, β-glucanase et cellulase. Cette étape a lieu en phase aqueuse à une concentration en produit sec comprise entre 7 % et 15% en poids. Le milieu réactionnel a été maintenu à une température de 60 °C +/- 10 °C et à un pH de 5 +/- 1,0 pendant 2 heures. Le rapport en poids enzyme / matière sèche de départ était de 0,1 %.

Puis on a séparé la phase liquide de la phase insoluble et on a séché chacune des fractions ainsi obtenues.

La fraction insoluble a été séchée sur cylindre et la phase liquide contenant la fraction soluble a été séchée par atomisation en utilisant une turbine ou une buse avec des températures en entrée de 210 °C +/-30 °C et en sortie de 100 °C +/- 10 °C.

### EXEMPLE 2 : Traitement du son de blé issu du procédé de meunerie

Les sons de blé étaient les mêmes que ceux utilisés à l'exemple 1 mais l'étape de traitement de l'amidon a été réalisée avec une α-amylase TERMAMYL 120 L^{®} de NOVO, en réacteur agité avec un rapport en poids enzyme / matière sèche de départ de 0,5 %.

La durée de la réaction du traitement de l'amidon était de 3 heures.

Quant à l'étape d'hydrolyse, elle a été réalisée avec l'enzyme SHEARZYME DOUGH^{®} de NOVO. L'enzyme SHEARZYME DOUGH^{®} est un enzyme ayant les activités enzymatiques xylanase, β-glucanase et cellulase. La durée de l'étape d'hydrolyse était de 3 heures. Le rapport en poids enzyme / matière sèche de départ était de 0,3 %.

Les autres conditions étaient les mêmes qu'à l'exemple 1.

### EXEMPLE 3

On a procédé comme à l'exemple 2 sauf que l'hydrolyse a été effectuée avec un enzyme FILTRASE BRX^{®} de DSM qui a les activités enzymatiques xylanase et β-glucanase et que le rapport en poids enzyme /matière sèche de départ était de 0,2 %.

### EXEMPLE 4

On a procédé comme à l'exemple 1 sauf que l'enzyme utilisé lors de l'hydrolyse était un enzyme NEUTRASE NL^{®} de DSM et que le rapport en poids enzyme / matière sèche de départ dans l'étape de séparation de l'amidon était de 1 %.

### EXEMPLE 5

On a procédé comme à l'exemple 1 sauf que l'enzyme PROTEASE NL^{®} de DSM a été utilisé lors de l'étape de l'hydrolyse.

### EXEMPLE 6

On a procédé comme à l'exemple 2 sauf que l'enzyme, RAPIDASE UF^{®} de DSM qui a les activités enzymatiques pectinase et hémicellulase a été utilisé lors de l'étape de l'hydrolyse.

### EXEMPLE 7 : Traitement du son de blé issu du procédé d'amidonnerie.

Du son de blé issu du procédé d'amidonnerie, ayant une granulométrie telle que 50 % des particules avaient un diamètre inférieur à 125 µm, a été utilisé.

Ce son de blé issu du procédé d'amidonnerie a été broyé en voie humide.

Après broyage, le son avait une granulométrie moyenne de 200 µm.

Ce son de blé contient environ 12,5 % en poids d'amidon par rapport à la matière humide, ce qui correspond à environ 50 % en poids d'amidon par rapport à la matière sèche.

En effet, le son de blé issu du procédé d'amidonnerie est obtenu sous forme humide.

On a alors procédé à l'étape de réduction de la teneur en amidon par action d'un enzyme α-amylase TERMAMYL 120 L^{®} de NOVO en jet cooker, qui permet un mélange efficace en continu d'un liquide, d'une suspension ou d'une pâte avec de la vapeur haute pression à une température de 105 °C +/- 5 °C pendant 10 min. Le rapport en poids enzyme / matière sèche de départ était de 0,8 %.

Puis l'amidon a été séparé du son de blé sur tamis conique.

Ensuite les étapes d'hydrolyse de séparation et de séchage ont été réalisées comme à l'exemple 1.

### EXEMPLE 8 : Traitement du son de blé issu du procédé d'amidonnerie

On a procédé comme à l'exemple 7 sauf que l'on a utilisé pour l'étape de réduction de la teneur en amidon, l'enzyme TERMAMYL 120 L^{®} de NOVO dans un réacteur en circulation avec échangeur de plaque à une température de 90 °C +/- 5 °C pendant 30 min et que le rapport en poids enzyme / matière sèche de départ était de 0,3 %.

On a également procédé comme pour le traitement du son de blé issu du procédé de meunerie, à des traitements de sons de blé issus du procédé d'amidonnerie en utilisant les mêmes conditions et enzymes, pour le traitement de l'amidon et pour l'hydrolyse, que décrits aux exemples qui précèdent.

L'étape d'hydrolyse dans tous les exemples de l'invention été réalisée en 2 à 3 heures, temps au bout duquel l'hydrolyse est optimale. Au-delà de 3 heures, l'hydrolyse est trop importante et produit trop de mono-saccharides.

Dans tous les cas, on a obtenu d'une part une fraction soluble et d'autre part une fraction insoluble avec un rendement pour cette étape, en fraction insoluble de 40 à 70 % en poids et en fraction soluble de 10 à 30 % en poids par rapport à la matière sèche de départ.

La capacité de rétention d'eau de la fraction insoluble obtenue est fortement améliorée par rapport à celle du son de blé de départ.

Les mesures de la capacité de rétention d'eau ont été réalisées dans les conditions suivantes :
Sur un échantillon de 1 g placé dans un tube à centrifuger, ajouter 18 g d'eau purifiée, laisser en contact pendant 16 h, centrifuger pendant 1 h à 2 700 tours/minute, égoutter sur une grille à mailles fines, pendant une durée < 10 s et peser le culot de centrifugation. Peser la masse d'eau non absorbée. En déduire la masse d'eau absorbée. Le résultat est exprimé en g d'eau absorbée par g d'échantillon.
Les résultats obtenus sont les suivants :

| Nature du produit testé | Capacité de rétention d'eau (WHC) en g d'eau/g de produit sec |
|---|---|
| Sons de blé de départ | 1,5 à 1,8 |
| Son de blé de départ micronisé | 2,0 |
| Produit insoluble provenant de l'exemple 1 | 5,5 |
| Produit insoluble provenant de l'exemple 2 | 6,0 |
| Produit insoluble provenant de l'exemple 7 | 5,5 |
| Produit insoluble provenant de l'exemple 8 | 6,0 |

De même la capacité de rétention d'huile de la fraction insoluble est améliorée par rapport à celle du son de départ.

Les mesures de capacité rétention d'huile ont été réalisées dans les conditions suivantes :
Sur un échantillon de 1 g placé dans un tube à centrifuger, ajouter 16 g d'huile de table commerciale, laisser en contact pendant 16 h, centrifuger pendant 1 h à 2 700 tours/min, égoutter sur une grille à masse fine, et peser le culot de centrifugation. Peser la masse d'huile non absorbée. En déduire la masse d'huile absorbée. Le résultat est exprimé en g d'huile absorbée par g de produit.

Les résultats obtenus sont les suivants :

| Nature du produit testé | Capacité de rétention en huile, en g d'huile/g de produit sec |
|---|---|
| Sons de blé de départ | 0,8 |
| Son de blé de départ micronisé | 1,8 |
| Produit insoluble provenant de l'exemple 7 | 2,5 |
| Produit insoluble provenant de l'exemple 8 | 2,5 |

Quant à la fraction soluble polysaccharidique, elle est enrichie en arabinoxylanes comme mesuré par chromatographie en phase gazeuse par la méthode ENGLYST. Dans cette méthode des étalons et des solutions témoins sont utilisées pour mesurer le facteur de réponse (FRP). Les échantillons et les témoins sont préparés selon le même protocole, extraction au solvant (chloroforme), acétylation, réduction, hydrolyse.

Cette fraction soluble a un rapport A/X compris entre 0,3 et 0,6, de préférence entre 0,33 et 0,46. Le rapport A/X a été déterminé par chromatographie en phase gazeuse, méthode ENGLYST, de chacune des concentrations en arabinose et en xylose. Le rapport A/X indique le taux de substitution de l'enchaînement xylose par arabinoses.

Cette fraction soluble contient des amyloses et des amylopectines de degrés de polymérisation supérieurs à 3 comme mesuré par HPLIC (Chromatographie Liquide Haute Performance Ionique et par CCM (Chromatographie sur Couche Mince) sur plaque de silice.

D'autres essais ont été réalisés sur du son provenant de maïs, de riz, de mil, de millet, de sorgho et de seigle. On a obtenu les mêmes avantages.

Des essais fructueux ont été réalisés sur des pois qui ne sont pas à proprement parler des céréales, mais qui sont considérés comme tels pour les buts de l'invention.

La fraction soluble issue du procédé de l'invention peut avantageusement être utilisée en industrie agroalimentaire, la parapharmacie et l'industrie cosmétique. Grâce au procédé de l'invention, la granulométrie peut être maîtrisée, par exemple par un broyage humide au cours de l'hydrolyse. La fraction soluble a également l'avantage de présenter une couleur blanche du produit sous forme pulvérulente et une couleur légèrement orangée à une concentration de 150 g/l dans de l'eau purifiée. La solubilité de la fraction soluble est totale à 400 g/l dans de l'eau purifiée à température ambiante. Une solution à 250 g/l dans de l'eau purifiée de la fraction soluble maintenue à 60°C est stable pendant 4 jours. La même observation a été faite lorsque le pH a été maintenu à pH 3 par ajout d'acide citrique. La méthode de suivi a été la mesure de la densité optique à 420 nm, pour la solution diluée 20 fois.

La viscosité de la fraction soluble est de 30 cps à une concentration de 25 % et de 128 cps à une concentration de 50 %. La viscosité est mesurée avec un viscosimètre Brookfield RVT à 25°C, mobile 2 et 50 tours/min.

La fraction insoluble quant à elle peut être également utilisée dans l'industrie agroalimentaire, la parapharmacie et l'industrie cosmétique ou dans des applications où la capacité de rétention d'eau et d'huile est une fonctionnalité recherchée ou encore en tant que produits satiétogènes.

La fraction insoluble présente l'avantage de ne contenir qu'une très faible quantité d'amidon, d'avoir un pouvoir de rétention en eau très amélioré par rapport à un son obtenu d'une façon classique en meunerie sèche et un pouvoir de rétention en huile également amélioré par rapport au son de départ.

La couleur de la fraction insoluble est claire.

La granulométrie peut être proche de celle de la farine classique : 80 % des particules passent au travers d'un tamis d'ouverture de maille inférieure à 250 µm.

Elle peut également trouver une application dans des applications de rétention d'eau dans des domaines variés, allant de la jardinerie jusqu'à l'isolation et aussi en tant que support d'arômes ou de parfum.

Les fractions solubles et insolubles peuvent avoir une application dans la conservation des produits congelés du fait de la présence d'arabinoxylanes, qui abaissent le point de congélation.

La fraction soluble peut être encapsulée éventuellement avec d'autres produits.

## Revendications

1. Procédé de traitement de son de blé issu du procédé de meunerie ou de son de blé issu du procédé d'amidonnerie, pour obtenir une fraction soluble dans l'eau et une fraction insoluble dans l'eau comprenant les étapes suivantes:
a) broyage du son à une granulométrie inférieure ou égale à 250 µm ; ensuite
b) réduction du taux d'amidon par action d'une enzyme α-amylase avec un rapport en poids enzyme/matière sèche de départ variant entre 0,1% et 1,0% ; puis,
c) hydrolyse enzymatique dans laquelle les enzymes utilisées ont au moins deux activités enzymatiques choisies parmi une activité xylanase et une activité cellulase, hémicellulase ou arabanase, où l'hydrolyse est réalisée à 60°C plus au moins 10 °C et à un pH de 5 plus ou moins 1,0 ; et où le rapport en poids enzyme/matière sèche de départ est compris entre 0,1 et 0,3% ; ensuite
d) séparation des fractions voulues, et
e) séchage des fractions séparées obtenues.

2. Procédé selon la revendication 1, **caractérisé en ce que** les enzymes utilisées à l'étape b) ont au moins les deux activités xylanase et cellulase.

3. Procédé selon la revendication 1, **caractérisé en ce que** les enzymes utilisées à l'étape b) ont au moins les deux activités xylanase et hémicellulase.

4. Procédé selon la revendication 1, **caractérisé en ce que** les enzymes utilisées à l'étape b) ont au moins les deux activités xylanase et arabanase.

5. Procédé selon l'une quelconque des revendications 1 à 4, **caractérisé en ce que** les sons sont les sons issus du procédé de meunerie.

6. Procédé selon l'une quelconque des revendications 1 à 4, **caractérisé en ce que** les sons sont les sons issus du procédé d'amidonnerie.

7. Fraction susceptible d'être obtenue par un procédé selon l'une des revendications 1 à 6 à partir d'un son de blé, **caractérisée en ce que**:
- elle est insoluble dans l'eau,
- elle a une teneur en amidon inférieure à 6 %,
- elle a une capacité de rétention d'eau supérieure ou égale à 5 g d'eau par g de fraction sèche et,
- une capacité de rétention d'huile mesurée supérieure ou égale à 2,0 g d'huile par g de fraction sèche.

8. Fraction susceptible d'être obtenue par un procédé selon l'une des revendications 1 à 6 à partir d'un son de blé, **caractérisée en ce que**:
- elle est soluble dans l'eau,
- elle a une teneur en arabinoxylanes supérieure ou égale à 30 % en poids par rapport à la masse sèche de la fraction totale, et **en ce que**
- les arabinoxylanes ont un rapport molaire arabinose/xylose noté A/X compris entre 0,33 et 0,46 ; et
- **caractérisée en ce que** les arabinoxylanes ont un degré de polymérisation, noté DP, supérieur ou égal à 3.

## Patentansprüche

1. Verfahren zur Behandlung von aus dem Mahlprozess stammender Weizenkleie oder von aus dem Stärkegewinnungsprozess stammender Weizenkleie zum Erhalt einer in Wasser löslichen Fraktion und einer in Wasser unlöslichen Fraktion, das die folgenden Schritte umfasst:
a) Zerkleinern der Kleie zu einer Korngröße kleiner oder gleich 250 µm; anschließend
b) Reduktion des Stärkeanteils durch Aktion eines Amylase α-enzyms mit einem Gewichtsanteil der Enzym-/Ausgangstrockensubstanz, der zwischen 0,1% und 1,0% schwankt; anschließend,
c) enzymatische Hydrolyse, bei der die eingesetzten Enzyme mindestens zwei Enzymaktivitäten haben, die aus einer Xylanase-Aktivität und einer Cellulase-, Hemicellulase- oder Arabanase-Aktivität ausgewählt wurden, wobei die Hydrolyse bei 60°C plus minus 10 °C und einem pH von 5 plus minus 1,0 durchgeführt wird; und wobei der Gewichtsanteil der Enzym/Ausgangstrockensubstanz zwischen 0,1 und 0,3% liegt; anschließend
d) Trennung der gewünschten Fraktionen, und
e) Trocknung der erhaltenen getrennten Fraktionen.

2. Verfahren nach Anspruch 1, **gekennzeichnet dadurch, dass** die in Schritt b) eingesetzten Enzyme mindestens die beiden Xylanase- und Cellulase-Aktivitäten besitzen.

3. Verfahren nach Anspruch 1, **gekennzeichnet dadurch, dass** die in Schritt b) eingesetzten Enzyme mindestens die beiden Xylanase- und Hemicellulase-Aktivitäten besitzen.

4. Verfahren nach Anspruch 1, **gekennzeichnet dadurch, dass** die in Schritt b) eingesetzten Enzyme mindestens die beiden Xylanase- und Arabanase-Aktivitäten besitzen.

5. Verfahren nach einem der Ansprüche 1 bis 4, **gekennzeichnet dadurch, dass** es sich bei den Kleien um die aus dem Mahlprozess stammenden Kleien handelt.

6. Verfahren nach einem der Ansprüche 1 bis 4, **gekennzeichnet dadurch, dass** es sich bei den Kleien um die aus dem Stärkegewinnungsprozess stammenden Kleien handelt.

7. Fraktion, die aus einem Verfahren nach einem der Ansprüche 1 bis 6 von einer Weizenkleie erhalten werden kann, **gekennzeichnet dadurch, dass**:
- sie in Wasser unlöslich ist,
- sie einen Stärkegehalt von weniger als 6 % aufweist,
- sie eine Wasserrückhaltefähigkeit größer oder gleich 5 g Wasser pro g Trockenanteil aufweist und
- eine gemessene Ölrückhaltefähigkeit größer oder gleich 2,0 g Öl pro g Trockenanteil besitzt.

8. Fraktion, die aus einem Verfahren nach einem der Ansprüche 1 bis 6 von einer Weizenkleie erhalten werden kann, **gekennzeichnet dadurch, dass**
- sie in Wasser löslich ist,
- sie einen Gehalt an Arabinoxylanen größer oder gleich 30 Gew.-% im Vergleich zur Trockenmasse des Gesamtanteils enthält, und dadurch, dass
- die Arabinoxylanen ein Arabinose/Xylose-Molverhältnis (A/X) haben, das zwischen 0,33 und 0,46 liegt; und
- **gekennzeichnet dadurch, dass** die Arabinoxylanen einen Polymerisationsgrad (DP) größer oder gleich 3 haben.

## Claims

1. Procedure for treatment of wheat bran made from a milling procedure or wheat bran made from a starch manufacturing procedure, to obtain a fraction which is soluble in water and a fraction which is insoluble in water comprising the following steps:
a) Crushing of the bran to a grain size less than or equal to 250 µm; then
b) reduction of the starch content by the action of an α-amylase enzyme with a weight ratio of enzyme/starting dry material varying between 0.1% and 1.0%; then,
c) enzymatic hydrolysis in which the enzymes used have at least two enzymatic activities chosen from a xylanase activity and cellulase, hemicellulase or arabanase activity whereby the hydrolysis is achieved at 60 °C plus or minus 10 °C and a pH of 5 plus or minus 1.0; and where the weight ratio of enzyme/starting dry material is between 0.1 and 0.3%; then
d) separation of the desired fractions, and
e) drying of the obtained separated fractions.

2. Procedure according to claim 1, **characterised in that** the enzymes used in step b) have at least two activities, xylanase and cellulase.

3. Procedure according to claim 1, **characterised in that** the enzymes used in step b) have at least two activities, xylanase and hemicellulase.

4. Procedure according to claim 1, **characterised in that** the enzymes used in step b) have at least two activities, xylanase and arabanase.

5. Procedure according to any one of claims 1 to 4, **characterised in that** the bran is bran issued from the milling procedure.

6. Procedure according to any one of claims 1 to 4, **characterised in that** the bran is bran issued from the starch manufacturing procedure.

7. Fraction which can be obtained from a procedure in accordance with one of claims 1 to 6 from a wheat bran, **characterised in that**:
- it is insoluble in water,
- it has a starch content of less than 6%,
- it has a water retention capacity more than or equal to 5 g of water per g of dry fraction, and
- an oil retention capacity measured of more than or equal to 2.0 g of oil per g of dry fraction.

8. Fraction which can be obtained from a procedure in accordance with one of claims 1 to 6 from a wheat bran, **characterised in that**:
- it is soluble in water,
- it has an arabinoxylan content greater than or equal to 30% in weight in relation to the dry mass of the total fraction, and **in that**
- the arabinoxylans have an arabinose/xylose molar ratio recorded as A/X between 0.33 and 0.46; and
- **characterised in that** the arabinoxylans have a degree of polymerisation, recorded as DP, greater than or equal to 3.
